# EUROPEAN PATENT APPLICATION

(11) **EP 4 159 255 A1**
(43) Date of publication of application: **05.04.2023**
(21) Application number: 22198104.6
(22) Date of filing: 27.09.2022
(51) Int. Cl.: A61M 5/142, A61M 39/22

(54) **DUAL NEEDLE VALVE FOR A WEARABLE DRUG DELIVERY DEVICE**

(30) Priority: 29.09.2021 US 202163249636 P
(71) Applicant: Insulet Corporation, Acton MA 01720 (US)
(72) Inventor: BREINGAN, Kyle, Lowell (US); CARDINALI, Steven, Tewksbury (US)
(74) Representative: Peterreins Schley

(57) **Abstract**

Disclosed is a valve for use in a pump for a wearable drug delivery device for moving a liquid drug from reservoir into a pump chamber and thereafter from the pump chamber to a patient interface. The valve can comprise two needles, a first needle in fluid communication with the reservoir and a second needle in fluid communication with the patient interface. The first and second needles may be linearly translated to either cover the lumen opening of a needle with a septum or place the lumen opening of the needle in fluid communication with the pump chamber. When the first needle is in fluid communication with the pump chamber, the liquid drug may be transferred from the reservoir to the pump chamber via suction created by a plunger in the pump chamber. When the second needle is in fluid communication with the pump chamber, the liquid drug may be transferred from the pump chamber to the patient interface via a pressure created by the plunger in the pump chamber.

## Description

### BACKGROUND

Many conventional drug delivery systems, in particular, systems which include a wearable drug delivery device, include a drug container within the wearable drug delivery device, often referred to as a reservoir, that stores a liquid drug for delivery to a user in accordance with an algorithm via a patient interface. The patient interface may be, for example, a needle and/or cannula which is inserted under the skin of the user.

A liquid drug stored in the reservoir may be transferred from the reservoir to the patient interface using a pump which draws the liquid drug from the reservoir into a pump chamber and expels the liquid drug from the pump chamber to the patient interface. One exemplary pump comprises a chamber having a driven plunger disposed therein which, when driven in one direction, cause a suction which draws the liquid drug from the reservoir into the pump chamber and, when driven in the opposite direction, pushes the liquid drug from the pump chamber to the patient interface. As the liquid drug is being drawn into the pump chamber, it is necessary to seal the pump chamber from the patient interface such as not to draw fluids from the patient into the pump chamber. Likewise, when expelling the drug from the pump chamber to the patient interface, it is necessary to seal the reservoir from the pump chamber to avoid returning the liquid drug to the reservoir instead of to the patient interface. This is typically accomplished via some form of valve.

### SUMMARY OF THE INVENTION

The exemplary embodiments of the invention described herein provide several variations of a new valve design suitable for use in a pump for a wearable drug delivery device. In a first embodiment of the invention, a needle connected to the reservoir and a needle connected to the patient interface are mechanically coupled to each other. The coupling causes the needles to move simultaneously in opposite directions. Movement of the needles in one direction will cause the patient interface needle to be pulled into a septum while the reservoir needle is pushed from a septum to allow fluid communication with the pump chamber. When the needles are moved in the opposite direction, the reservoir needle is pulled into the septum while the patient interface needle is pushed from the septum to allow fluid communication with the pump chamber. In this embodiment, the needles are pulled into a septa to seal the needle from the pump chamber. In a variation of this embodiment, the needles may be pushed into a septa (as opposed to being pulled into a septa) to seal the needles from the pump chamber.

In a second embodiment of the invention, the reservoir needle and patient interface needle are mechanically coupled but move in the same direction. Movement of the pair of needles in one direction pushes the patient interface needle into a septum and brings the reservoir needle into fluid communication with the pump chamber. Movement of the pair of needles in the opposite direction pulls the reservoir needle into a septum and brings the patient interface needle into fluid communication with the pump chamber. In a variation of this embodiment, the needles are configured in the opposite direction such that the reservoir needle is pushed into the septum to create a seal and the patient interface needle is pulled into the septum to create the seal.

In a third embodiment of the invention, the needles are not coupled to each other but instead may be moved independently. Each needle may be pushed or pulled independent of the other needle so as to position it in a septum to seal it from the pump chamber or allow it to be a fluid communication with the pump chamber. In this embodiment, it is possible that both the reservoir needle and the patient interface needle will be in fluid communication with the pump chamber at the same time. This "open-open" valve solution allows the use of gaseous sterilization techniques to sterilize the pump and helps with priming the pump and clearing the air from the fluid path between the reservoir and the patient interface during the priming stage.

### BRIEF DESCRIPTION OF THE DRAWINGS

**FIG. 1** illustrates a functional block diagram of an exemplary system suitable for implementing the systems and methods disclosed herein.
**FIGS. 2****(A-B)** are cross-sectional schematic diagrams showing a first embodiment of the invention.
**FIGS. 3****(A-B)** are cross-sectional schematic diagrams showing a variation of the embodiment of **FIGS. 2****(A-B).**
**FIGS. 4****(A-B)** are cross-sectional schematic diagrams showing a second embodiment of the invention.
**FIGS. 5****(A-C)** are cross-sectional schematic diagrams showing a third embodiment of the invention.
**FIG. 6** is a diagram showing one of three possible states of various embodiments of the invention.

### DETAILED DESCRIPTION

This disclosure presents various systems, components and methods for moving a liquid drug from a liquid reservoir in a wearable drug delivery device 100 to a patient interface, typically a needle or cannula. The embodiments described herein provide one or more advantages over conventional, prior art systems, components and methods, namely, a smaller footprint and reduced energy consumption. The novel aspects of the embodiments of the present invention are described in detail below. Several exemplary embodiments are shown herein; however, it should be realized that invention is not meant to be limited thereby but is instead meant to encompass the novel aspects of the various embodiments.

Various embodiments of the present invention include systems and methods for delivering a medication to a user using a wearable drug device (sometimes referred to herein as a "pod"), either autonomously, or in accordance with a wireless signal received from an electronic device. In various embodiments, the electronic device may be a user device comprising a smartphone, a smart watch, a smart necklace, a module attached to the drug delivery device, or any other type or sort of electronic device that may be worn or carried on the body of the user and that executes an algorithm that computes the times and dosages of delivery of the medication. For example, the user device may execute an "artificial-pancreas" algorithm that computes the times and dosages of delivery of insulin. The user device may also be in communication with a sensor, such as a glucose sensor, that collects data on a physical attribute or condition of the user, such as a glucose level. The sensor may be disposed in or on the body of the user and may be part of the drug delivery device or may be a separate device. Alternately, the drug delivery device may be in communication with the sensor in lieu of or in addition to the communication between the sensor and the user device. The communication may be direct (if, e.g., the sensor is integrated with or otherwise a part of the drug delivery device) or remote/wireless (if, e.g., the sensor is disposed in a different housing than the medical device). In these embodiments, the sensor and/or drug delivery device contain computing hardware (e.g., a processor, memory, firmware, etc.) that executes some or all of the algorithm that computes the times and dosages of delivery of the medication.

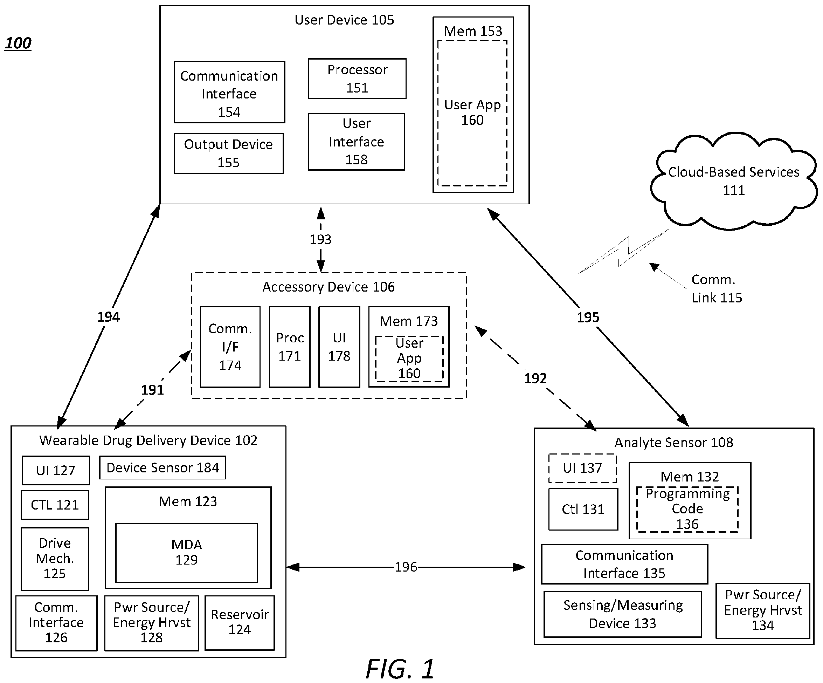

**FIG. 1** illustrates a functional block diagram of an exemplary system suitable for implementing the systems and, methods described herein. The automatic drug delivery system 100 may implement (and/or provide functionality for) a medication delivery algorithm, such as an artificial pancreas (AP) application, to govern or control automated delivery of a drug or medication, such as insulin, to a user (e.g., to maintain euglycemia - a normal level of glucose in the blood). The drug delivery system 100 may be an automated drug delivery system that may include a wearable drug delivery device 102, an analyte sensor 108, and a user device 105.

The system 100, in an optional example, may also include an accessory device 106, such as a smartwatch, a personal assistant device or the like, which may communicate with the other components of system 100 via either a wired or wireless communication links 191-193.

The user device 105 may be a computing device such as a smartphone, a tablet, a personal diabetes management (PDM) device, a dedicated diabetes therapy management device, or the like. In an example, user device 105 may include a processor 151, device memory 153, a user interface 158, and a communication interface 154. The user device 105 may also contain analog and/or digital circuitry that may be implemented as a processor 151 for executing processes based on programming code stored in device memory 153, such as user application 160 to manage a user's blood glucose levels and for controlling the delivery of the drug, medication, or therapeutic agent to the user, as well for providing other functions, such as calculating carbohydrate-compensation dosage, a correction bolus dosage and the like as discussed below. The user device 105 may be used to program, adjust settings, and/or control operation of the wearable automatic drug delivery device 102 and/or the analyte sensor 103 as well as the optional smart accessory device 106.

The processor 151 may also be configured to execute programming code stored in device memory 153, such as the user app 160. The user app 160 may be a computer application that is operable to deliver a drug based on information received from the analyte sensor 103, the cloud-based services 111 and/or the user device 105 or optional accessory device 107. The memory 153 may also store programming code to, for example, operate the user interface 158 (e.g., a touchscreen device, a camera or the like), the communication interface 154 and the like. The processor 151, when executing user app 160, may be configured to implement indications and notifications related to meal ingestion, blood glucose measurements, and the like. The user interface 158 may be under the control of the processor 151 and be configured to present a graphical user interface that enables the input of a meal announcement, adjust setting selections and the like as described herein.

In a specific example, when the user app 160 is an artificial pancreas (AP) application, the processor 151 is also configured to execute a diabetes treatment plan (which may be stored in a memory) that is managed by user app 160. In addition to the functions mentioned above, when user app 160 is an AP application, it may further provide functionality to determine a carbohydrate-compensation dosage, a correction bolus dosage and determine a basal dosage according to a diabetes treatment plan. In addition, as an AP application, user app 160 provides functionality to output signals to the wearable automatic drug delivery device 102 via communications interface 154 to deliver the determined bolus and basal dosages.

The communication interface 154 may include one or more transceivers that operate according to one or more radio-frequency protocols. In one embodiment, the transceivers may comprise a cellular transceiver and a Bluetooth^{®} transceiver. The communication interface 154 may be configured to receive and transmit signals containing information usable by user app 160.

User device 105 may be further provided with one or more output devices 155 which may be, for example, a speaker or a vibration transducer, to provide various signals to the user.

The wearable automatic drug delivery device 102, in the example system 100, may include a user interface 127, a controller 121, a drive mechanism 125, a communication interface 126, a memory 623, a power source/energy harvesting circuit 128, device sensors 184, and a reservoir 124. The wearable automatic drug delivery device 102 may be configured to perform and execute processes required to deliver doses of the medication to the user without input from the user device 105 or the optional accessory device 106. As explained in more detail, the controller 121 may be operable, for example, to determine an amount of insulin to be delivered, IOB, insulin remaining, and the like, based on an input from the analyte sensor 108.

The memory 123 may store programming code executable by the controller 121. The programming code, for example, may enable the controller 121 to control the delivery of medication from the reservoir 124 and control the administering of doses of medication based on signals from the medication delivery algorithm (MDA) 129 or, external devices, if the MDA 129 is configured to implement the external control signals.

The reservoir 124 may be configured to store drugs, medications or therapeutic agents suitable for automated delivery, such as insulin, GLP-1, co-formulations of insulin and GLP-1, morphine, blood pressure medicines, chemotherapy drugs, fertility drugs or the like.

The device sensors 184 may include one or more of a pressure sensor, a power sensor, or the like that are communicatively coupled to the controller 121 and provide various signals. For example, a pressure sensor may be configured to provide an indication of the fluid pressure detected in a fluid pathway between a needle or cannula inserted in a user and the reservoir 124. The pressure sensor may be coupled to or integral with a needle/cannula insertion component (which may be part of the drive mechanism 125) or the like. In an example, the controller 121 or a processor, such as 151, may be operable to determine that a rate of drug infusion based on the indication of the fluid pressure. The rate of drug infusion may be compared to an infusion rate threshold, and the comparison result may be usable in determining an amount of insulin onboard (IOB) or a total daily insulin (TDI) amount.

In an example, the wearable automatic drug delivery device 102 includes a communication interface 126, which may be a transceiver that operates according to one or more radio-frequency protocols, such as Bluetooth, Wi-Fi, near-field communication, cellular, or the like. The controller 121 may, for example, communicate with user device 105 and an analyte sensor 108 via the communication interface 126.

The wearable automatic drug delivery device 102 may be attached to the body of a user, such as a patient or diabetic, at an attachment location and may deliver any therapeutic agent, including any drug or medicine, such as insulin or the like, to a user at or around the attachment location. A surface of the wearable automatic drug delivery device 102 may include an adhesive to facilitate attachment to the skin of a user.

The wearable automatic drug delivery device 102 may, for example, include a reservoir 124 for storing the drug, a needle or cannula (not shown) for delivering the drug into the body of the user (which may be done subcutaneously, intraperitoneally, or intravenously), and a drive mechanism 125 for transferring the drug from the reservoir 124 through a needle or cannula and into the user. The drive mechanism 125 may be fluidly coupled to reservoir 124, and communicatively coupled to the controller 121.

The wearable automatic drug delivery device 102 may further include a power source 128, such as a battery, a piezoelectric device, an energy harvesting devices, or the like, for supplying electrical power to the drive mechanism 125 and/or other components (such as the controller 121, memory 123, and the communication interface 126) of the wearable automatic drug delivery device 102.

In some examples, the wearable automatic drug delivery device 102 and/or the user device 105 may include a user interface 158, and an output device 155, such as a keypad, a touchscreen display, levers, light-emitting diodes, buttons on a housing of the drug delivery device 101, a microphone, a camera, a speaker, a display, or the like, that is configured to allow a user to enter information and allow the user device 105 to output information for presentation to the user (e.g., alarm signals or the like). The user interface 158 may provide inputs, such as a voice input, a gesture (e.g., hand or facial) input to a camera, swipes to a touchscreen, or the like, to processor 151 which the user app 160 interprets.

When configured to communicate with an external device, such as the user device 105 or the analyte sensor 108, the wearable automatic drug delivery device 102 may receive signals over the wired or wireless link 194 from the user device 105 or from the analyte sensor 108. The controller 121 of the wearable automatic drug delivery device 102 may receive and process the signals from the respective external devices as well as implementing delivery of a drug to the user according to a diabetes treatment plan or other drug delivery regimen.

In an operational example, the processor 121, when executing user app 160, may output a control signal operable to actuate the drive mechanism 125 to deliver a carbohydrate-compensation dosage of insulin, a correction bolus, a revised basal dosage or the like.

The accessory device 107 may be, for example, an Apple Watch^{®}, other wearable smart device, including eyeglasses, smart jewelry, a global positioning system-enabled wearable, a wearable fitness device, smart clothing, or the like. Similar to user device 105, the accessory device 107 may also be configured to perform various functions including controlling the wearable automatic drug delivery device 102. For example, the accessory device 107 may include a communication interface 174, a processor 171, a user interface 178 and a memory 173. The user interface 178 may be a graphical user interface presented on a touchscreen display of the smart accessory device 107. The memory 173 may store programming code to operate different functions of the smart accessory device 107 as well as an instance of the user app 160, or a pared-down versions of user app 160 with reduced functionality.

The analyte sensor 108 may include a controller 131, a memory 132, a sensing/measuring device 133, an optional user interface 137, a power source/energy harvesting circuitry 134, and a communication interface 135. The analyte sensor 603 may be communicatively coupled to the processor 651 of the management device 605 or controller 621 of the wearable automatic drug delivery device 602. The memory 632 may be configured to store information and programming code 136.

The analyte sensor 108 may be configured to detect multiple different analytes, such as lactate, ketones, uric acid, sodium, potassium, alcohol levels or the like, and output results of the detections, such as measurement values or the like. The analyte sensor 108 may, in an exemplar embodiment, be configured to measure a blood glucose value at a predetermined time interval, such as every 5 minutes, or the like. The communication interface 135 of analyte sensor 108 may have circuitry that operates as a transceiver for communicating the measured blood glucose values to the user device 105 over a wireless link 195 or with wearable automatic drug delivery device 102 over the wireless communication link 108. While referred to herein as an analyte sensor 108, the sensing/measuring device 133 of the analyte sensor 108 may include one or more additional sensing elements, such as a glucose measurement element, a heart rate monitor, a pressure sensor, or the like. The controller 131 may include discrete, specialized logic and/or components, an applicationspecific integrated circuit, a microcontroller or processor that executes software instructions, firmware, programming instructions stored in memory (such as memory 132), or any combination thereof.

Similar to the controller 121 of drug delivery device 102, the controller 131 of the analyte sensor 108 may be operable to perform many functions. For example, the controller 131 may be configured by programming code 136 to manage the collection and analysis of data detected by the sensing and measuring device 133.

Although the analyte sensor 108 is depicted in **FIG. 1** as separate from the wearable automatic drug delivery device 102, in various examples, the analyte sensor 108 and wearable automatic drug delivery device 102 may be incorporated into the same unit. That is, in various examples, the analyte sensor 108 may be a part of an integral with the wearable automatic drug delivery device 102 and contained within the same housing as the wearable automatic drug delivery device 102. In such an example configuration, the controller 121 may be able to implement the functions required for the proper delivery of the medication alone without any external inputs from user device 105, the cloud-based services 111, another sensor (not shown), the optional accessory device 107, or the like.

The communication link 115 that couples the cloud-based services 111 to the respective devices 102, 105, 106, 108 of system 100 may be a cellular link, a Wi-Fi link, a Bluetooth link, or a combination thereof. Services provided by cloud-based services 111 may include data storage that stores anonymized data, such as blood glucose measurement values, historical IOB or TDI, prior carbohydrate-compensation dosage, and other forms of data. In addition, the cloud-based services 111 may process the anonymized data from multiple users to provide generalized information related to TDI, insulin sensitivity, IOB and the like.

The wireless communication links 191-196 may be any type of wireless link operating using known wireless communication standards or proprietary standards. As an example, the wireless communication links 191-196 may provide communication links based on Bluetooth^{®}, Zigbee^{®}, Wi-Fi, a near-field communication standard, a cellular standard, or any other wireless protocol via the respective communication interfaces 154, 174, 126 and 135.

The user app 160 (or MDA 129) may provide periodic insulin micro-boluses based upon the predicted glucose over a 60-minute prediction horizon. Optimal post-prandial control will require the user to give meal boluses in the same manner as current pump therapy, but normal operation of the user app 160 will compensate for missed meal boluses and mitigate prolonged hyperglycemia. The user app 160 uses a control-to-target strategy that attempts to achieve and maintain a set target glucose value, thereby reducing the duration of prolonged hyperglycemia and hypoglycemia.

The user application 160 implements a graphical user interface that is the primary interface with the user and is used to start and stop a wearable drug delivery device 102, program basal and bolus calculator settings for manual mode as well as program settings specific for automated mode (hybrid closed-loop or closed-loop).

In manual mode, user app 160 will deliver insulin at programmed basal rates and bolus amounts with the option to set temporary basal profiles. The controller 121 will also have the ability to function as a sensor-augmented pump in manual mode, using sensor glucose data provided by the analyte sensor 108 to populate the bolus calculator.

In automated mode, the user app 160 supports the use of multiple target blood glucose values. For example, in one embodiment, target blood glucose values can range from 110-150 mg/dL, in 10 mg/dL increments, in 5 mg/dL increments, or other increments, but preferably 10 mg/dL increments. The experience for the user will reflect current setup flows whereby the healthcare provider assists the user to program basal rates, glucose targets and bolus calculator settings. These in turn will inform the user app 160 for insulin dosing parameters. The insulin dosing parameters will be adapted over time based on the total daily insulin (TDI) delivered during each use of drug delivery device 102. A temporary hypoglycemia protection mode may be implemented by the user for various time durations in automated mode. With hypoglycemia protection mode, the algorithm reduces insulin delivery and is intended for use over temporary durations when insulin sensitivity is expected to be higher, such as during exercise.

User app 160, allows the use of large text, graphics, and on-screen instructions to prompt the user through the set-up processes and the use of system 100. It will also be used to program the user's custom basal insulin delivery profile, check the status, of drug delivery device 102, initiate bolus doses of insulin, make changes to a patient's insulin delivery profile, handle system alerts and alarms, and allow the user to switch between automated mode and manual mode.

In some embodiments, user device 105 and the analyte sensor 108 may not communicate directly with one another. Instead, data (e.g., blood glucose readings) from analyte sensor may be communicated to drug delivery device 102 via link 196 and the relayed top user device 102 via link 194. In some embodiments, to enable communication between analyte sensor 108 and user device 102, the serial number of the analyte sensor must be entered into user app 160.

User ap 160 may provide the ability to calculate a suggested bolus dose through the use of a bolus calculator. The bolus calculator is provided as a convenience to the user to aid in determining the suggested bolus dose based on ingested carbohydrates, most-recent blood glucose readings (or a blood glucose reading if using fingerstick), programmable correction factor, insulin to carbohydrate ratio, target glucose value and insulin on board (IOB). IOB is estimated by user app 160 taking into account any manual bolus and insulin delivered by the algorithm.

Software related implementations of the techniques described herein may include, but are not limited to, firmware, application specific software, or any other type of computer readable instructions that may be executed by one or more processors. The computer readable instructions may be provided via non-transitory computer-readable media. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

The primary embodiments of the invention are directed to a valve suitable for use in a pump mechanism of a wearable drug delivery device. The pump mechanism comprises a pump chamber having disposed therein a plunger which, when moved in one direction, creates suction within the pump chamber and, when moved in the other direction creates a pressure within the pump chamber. The plunger may be moved in either direction within the pump chamber via any one of a number of well-known mechanisms, including, for example, via a direct connection to a leadscrew, via a connection to a mechanical linkage driven by a motor, via a connection to a flexible cable which is pulled through the pump chamber, and via a connection to a device composed of a shape memory alloy (SMA) such as Nitinol. Both the pump chamber and the plunger may be composed of a hard plastic material such as polyethylene. The plunger may be fitted with one or more O-rings composed of rubber or silicone around an outside perimeter so as to allow movement of the plunger within the pump chamber while at same time preventing leakage of the liquid drug past the plunger.

The pump chamber is in fluid communication with both a reservoir and a patient interface via respective needles which are inserted into the pump chamber through a septum. The needles may be connected to the reservoir and the patient interface via a flexible conduit. The reservoir may be a rigid structure composed of a plastic such as polyethylene, or may be a collapsible structure composed of a flexible material which is impermeable or semi-impermeable to air.

In embodiments of the invention described herein, seals are created by movement of needles such that an opening to the lumen of the needle is disposed within a septum. In various embodiments described herein, the septa used for this purpose may be external to pump chamber 108, internal to pump chamber 108, or both. The septa may be composed of, for example, rubber or silicon.

The various embodiments of the invention discussed below explain in detail various ways of enabling or restricting fluid communication between the pump chamber and either or both of the reservoir and patient interface.

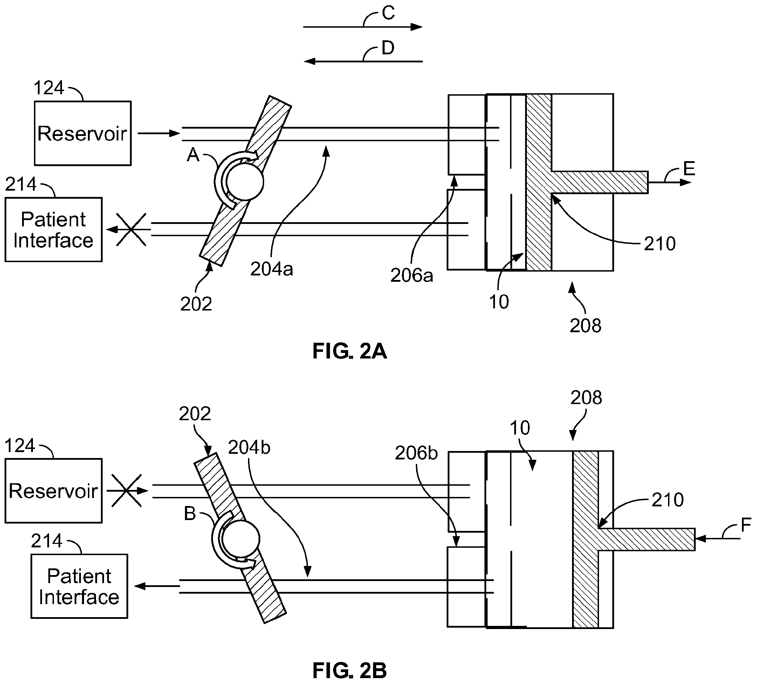

**FIGS. 2****(A-B)** show cross-sectional views of a first embodiment of the invention. In this embodiment of the invention, pump chamber 208 is either in fluid communication with a reservoir 124 containing a liquid drug 10 via needle 204a or in fluid communication with a patient interface 214 via needle 204b. Needles 204a, 204b are mechanically coupled to each other via actuator 202. In preferred embodiments of the invention, actuator 202 may be rotationally translated in opposite directions as shown by arrows **A** and **B** in **FIGS. 2****(A-B)** so as to linearly translate needles 204a, 204b in opposite directions. While actuator 202 is shown as being rotationally translatable, any design for actuator 202 capable of moving needles 204a, 204b simultaneously in opposite linear directions may be used. Actuator 202 may be rotated or otherwise moved by any known means. In preferred embodiments, actuator 202 may comprise one or more shape memory alloy wires and/or one or more springs. Actuator 202 may be connected to needles 204a, 204b by any known means.

**FIG. 2A** shows a first state of this embodiment of the valve in which pump chamber 208 is in fluid communication with the reservoir 124. In this state, actuator 202 has been rotated in direction **A,** which causes needle 204a to be linearly translated in direction **C** and, as a result, pushed out of septum 206a and into fluid communication with pump chamber 208. At the same time, needle 204b is linearly translated in direction **D** so as to be removed from fluid communication with pump chamber 208 by being pulled into septum 206b, thereby creating a seal between pump chamber 208 and the patient interface 214. In this state, movement of plunger 210 in direction **E,** will cause liquid drug 10 to be drawn from reservoir 124 into pump chamber 208 via needle 204a. As indicated by the "X" in **FIG. 2A****,** patient interface 214 will be sealed from pump chamber 208 so as to prevent fluids from being drawn from the patient interface 214 into pump chamber 208 as a result of the suction caused by movement of plunger 210 in direction **E.** Note that, in this embodiment, septa 206a, 206b are exterior to pump chamber 208 and also serve to create a seal around needles 204a, 204b to prevent leakage of liquid drug 10 from pump chamber 208. While septa 206a, 206b are shown and described herein as being separate, in practice they may be implemented as a single septum.

**FIG. 2B** shows a second state of this embodiment of the valve in which pump chamber 208 is in fluid communication with the patient interface 214. In this state, actuator 202 has been rotated in direction **B,** which causes needle 204a to be linearly translated in direction **D** and, as a result, removed from fluid communication with pump chamber 208 by being pulled into septum 206a, thereby creating a seal between pump chamber 208 and reservoir 12. At the same time, needle 204b is linearly translated in linear direction **C** so as to be pushed out of septum 206b and placed in fluid communication with pump chamber 208, thereby placing pump chamber 208 in fluid communication with patient interface 214. Preferably, the state shown in **FIG. 2B** will follow the state shown in **FIG. 2A** such that pump chamber 208 is filled with liquid drug 10 prior to rotation of actuator 202 in direction **B** to set the valve for delivery of the liquid drug 10 to patient interface 214. In this state, movement of plunger 210 in direction **F,** will cause liquid drug 10 to be pushed from pump chamber 208 to patient interface 214 via the pressure created by the movement of plunger 210 in direction **F.** As indicated by the "X" in **FIG. 2B****,** reservoir 12 will be sealed from pump chamber 208 so as to prevent liquid drug 10 from being pushed from pump chamber 208 into reservoir 12 as a result of the pressure caused by movement of plunger 210 in direction **F.**

Plunger 210 may be moved in directions **E** or **F** by any known means. In preferred embodiment, the means of movement of plunger 210 may comprise a combination of an SMA wire and a spring, or a combination of two SMA wires. In some embodiments, directions **C** and **E** indicate the same direction and directions **D** and **F** indicate the same direction, opposite directions **C** and **E.**

In preferred embodiments, it is preferable that the septa 206a, 206b be large enough such that the openings to the lumina of both of needles 204a, 204b may be contained within septa 206a, 206b at the same time as they are moved in opposite directions. That is, it is preferable that the opening of the lumen of one needle be contained within a septum before the opening of the lumen of the other needle exits the septum, thereby avoiding an "open-open" scenario in which both needles 204a, 204b are in fluid communication with the pump chamber 208 at the same time.

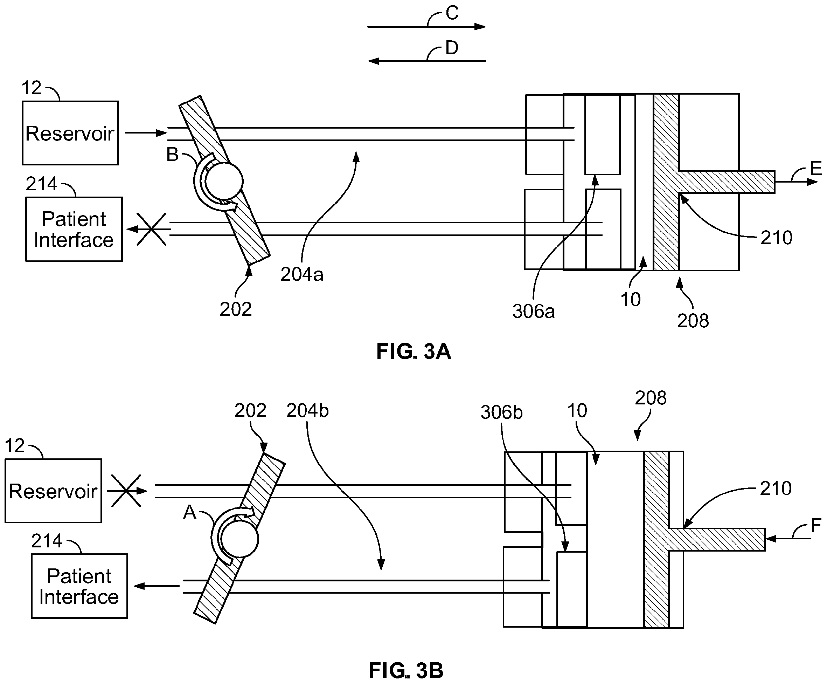

**FIGS. 3****(A-B)** show a variation of the embodiment of **FIGS. 2****(A-B).** In this variation, interior septa 306a, 306b have been added. As with the previous embodiment, septa 206a, 206b create a seal around needles 204a, 204b to prevent liquid drug 10 from leaking from pump chamber 208. Septa 306a, 306b are used to seal the openings of the lumina of needles 204a and 204b respectively from pump chamber 208. Note that, while septa 306a, 306b are shown and described herein as being separate, in practice they may be implemented as a single septum with a central hole therein to allow liquid drug 10 to pass therethrough.

**FIG. 3A** shows a first state of this embodiment of the valve wherein reservoir 12 is in fluid communication with pump chamber 208 via needle 204a and patient interface 214 is sealed from pump chamber 208. When actuator 202 is rotated in direction **B,** needle 204a is linearly translated in direction **D** so as to be pulled from septum 306a and placed into fluid communication with pump chamber 208. At the same time, needle 204b is linearly translated in direction **C** so as to be pushed into septum 306b, thereby removing it from fluid communication with pump chamber 208 and sealing patient interface 214 from pump chamber 208, as shown by the "X" in **FIG. 3A****.** In this state, movement of plunger 210 in direction **E** causes liquid drug 10 to be drawn from reservoir 12 and into pump chamber 208, while preventing fluids from patient interface 214 from entering pump chamber 208.

**FIG. 3B** shows a second state of this embodiment of the valve wherein patient interface 214 is in fluid communication with pump chamber 208 via needle 204b. When actuator 202 is rotated in direction **A,** needle 204a is linearly translated in direction **C** so as to be pushed into septum 306a, thereby removing it from fluid communication with pump chamber 208 and sealing reservoir 12 from pump chamber 208, as shown by the "X" in **FIG. 3B****.** At the same time, needle 204b is linearly translated in direction **D** so as to be pulled from septum 306b and placed in fluid communication with pump chamber 208. Preferably, the state shown in **FIG. 3B** will follow the state shown in **FIG. 3A** such that pump chamber 208 is filled with liquid drug 12 prior to rotation of coupling 202 in direction **A** to set up the device for delivery of liquid drug 12 to patient interface 214. When plunger 210 is moved in direction **F,** liquid drug 10 is forced from pump chamber 208 into patient interface 214, while liquid drug 210 is prevented from returning to reservoir 12. As with the first embodiment, it is preferable that the septa 306a, 306b be large enough to allow the openings of the lumina of both needles 204a, 204b to be contained within a septum as they are linearly translated to prevent the "open-open" situation.

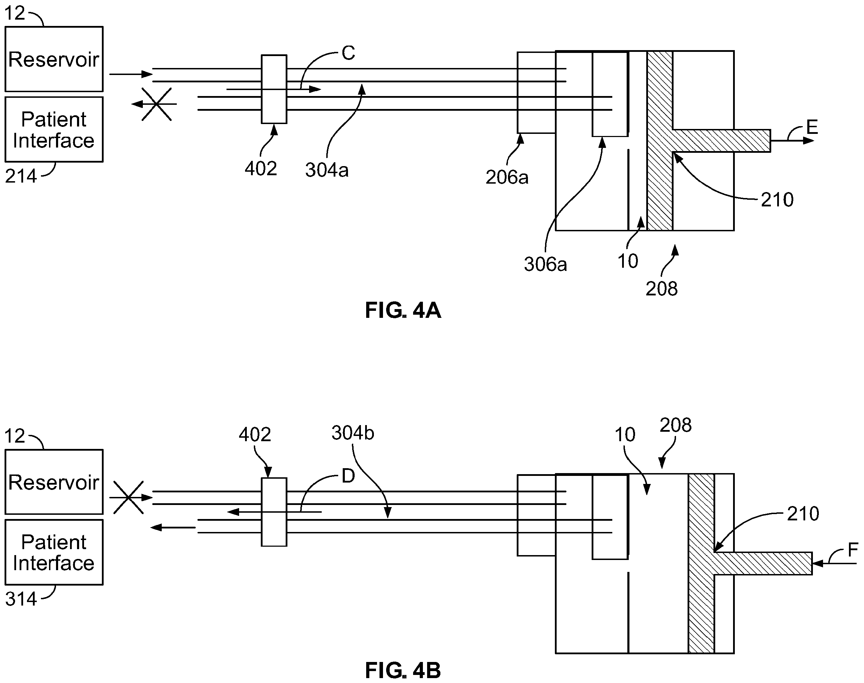

**FIGS. 4****(A-B)** shows a second embodiment of the invention in which actuator 402 mechanically couples needles 204a, 204b. Actuator 402, when linearly translated in either direction **C** or **D** also linearly translates both needles 204a, 204b in the same direction. Needles 204a, 204b are preferably either of different lengths or are offset from one another with respect to pump chamber 208. Note that actuator 402 may be of any design and may be linearly translated in either direction **C** or **D** via any known means. In preferred implementations of this embodiment of the invention, actuator 402 may comprise one or more shape memory alloy wires and/or one or more springs. In this embodiment of the invention, a dual lumen needle could be used in lieu of separate needles 204a, 204b, wherein the openings of the lumina are offset such that one lumen opening is sealed by a septum while the other lumen opening is in fluid communication with pump chamber 210.

**FIG. 4A** shows a first state of this embodiment of the valve wherein linear translation of actuator 402 in direction **C** causes both needles 204a, 204b to also move in direction **C.** Based on this movement, needle 204a is pushed out of exterior septum 206a and placed in fluid communication with pump chamber 208. At the same time, needle 204b is pushed into interior septum 306a, thereby removing it from fluid communication with pump chamber 208 and sealing patient interface 214 from pump chamber 108, as indicated by the "X" in **FIG. 4A****.** In this state, movement of plunger 210 in direction **E** will create a suction which causes liquid drug 10 in reservoir 12 to be drawn into pump chamber 208. Note that exterior septum 206a also serves to create a seal around both needles 204a, 204b, thereby preventing leakage of liquid drug 10 from pump chamber 208.

**FIG. 4B** shows a second state of this embodiment of the valve wherein a linear translation of actuator 402 in direction **D** causes both needles 204a, 204b to also move in direction **D.** Based on this movement, the opening of the lumen of needle 204a is pulled into exterior septum 206a, thereby removing it from fluid communication with pump chamber 208 and sealing reservoir 12 from pump chamber 208, as shown by the "X" in **FIG. 4B****.** At the same time, needle 204b is pulled from interior septum 306a, thereby placing it in fluid communication with pump chamber 208 and allowing movement of liquid drug 10 to patient interface 214. Preferably, the state shown in **FIG. 4B** follows the state shown in **FIG. 4A** such that pump chamber 208 is filled with liquid drug 10 prior to movement of the needles 204a, 204b in direction **D** to set up the device for delivery of the liquid drug 10 to patient interface 214. When plunger 210 is moved in direction **F,** liquid drug 10 is forced from pump chamber 208 to patient interface 214 via needle 204b. As with the other embodiments so far discussed, it is preferable that the "open-open" situation be avoided. In this case, the offsets of the openings of the lumina of the needles with respect to each other (or the difference in length of the needles if the lumen openings are at the ends of the needles) must be large enough to allow one needle to be pushed or pulled into a septum before the other needle is pushed or pulled out of a septum. However, in an alternative variation, the lumen openings of the needles could be offset such that an "open-open" situation is possible, so as to enable sterilization of the pump components, for example. In such a case, plunger 210 would preferably not be moved when the needles are in the "open-open" orientation. A similar alternative variation is possible for the examples shown in **FIGS. 2A-3B****.**

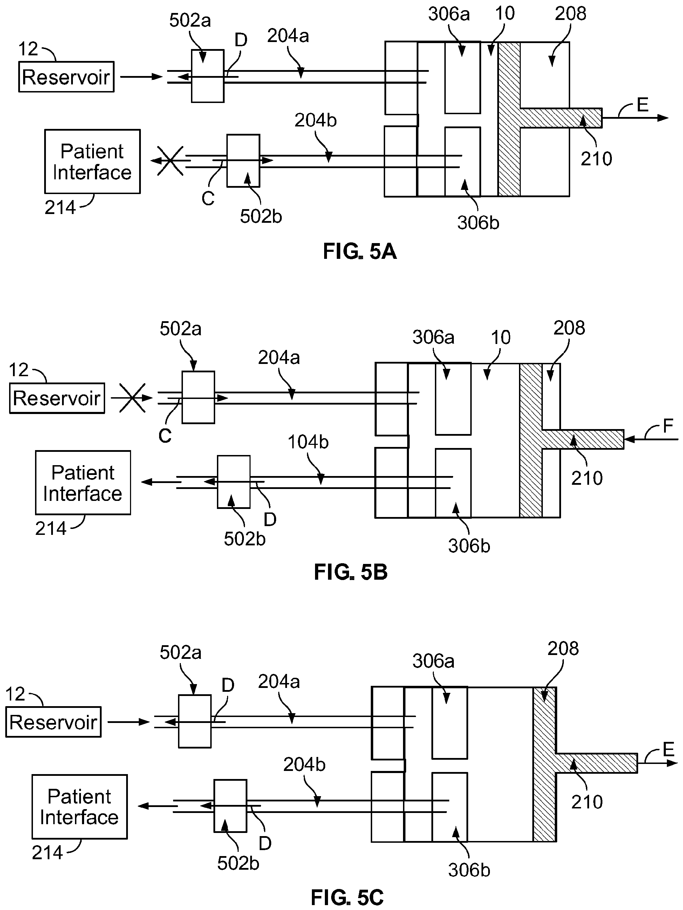

**FIGS. 5****(A-C)** show a third embodiment of the valve in which needles 204a, 204b are not mechanically coupled but may be linearly translated in either direction **C** or **D** independent of each other. Needles 204a, 204b may be linearly translated in either direction by actuators 502a, 502b respectively. Actuators 502a, 502b may be of any design and may accomplish the translation of needles 204a, 204b in either direction **C** or **D** by any known means. In preferred implementations of this embodiment, actuators 502a, 502b may comprise one or more shape memory alloy wires and/or one or more springs.

This embodiment uses an arrangement of septa similar to the embodiment shown in **FIGS. 3****(A-B)** wherein exterior septa 206a, 206b create seals around needles 204a, 204b to prevent leakage of liquid drug 10 from pump chamber 208, and further wherein interior septa 306a, 306b serve to seal needles 204a, 204b from pump chamber 208 when their respective lumina are disposed within either septa 306a or 306b.

**FIG. 5A** shows a first state of this embodiment of the valve wherein pump chamber 208 is in fluid communication with reservoir 12 via needle 204a and patient interface 214 is sealed from pump chamber 208, as shown by the "X" in **FIG. 5A****.** In this state, needle 204a has been linearly translated in direction **D** so as to pull needle 204a from septum 306a. Independently, needle 204b has been linearly translated in direction **C** to push it into septum 306b, thereby sealing needle 204b from pump chamber 208. In this embodiment, movement of plunger 210 in direction **E** causes a suction in pump chamber 208 and causes liquid drug 10 to be drawn from reservoir 12 and into pump chamber 208, while preventing fluids from entering pump chamber 208 from patient interface 214.

**FIG. 5B** shows a second state of this embodiment of the valve wherein pump chamber 208 is in fluid communication with patient interface 214 via needle 204b. In this state, needle 204b is moved in direction **D,** pulling it from septum 306b and placing it in fluid communication with pump chamber 208. Independently, needle 204a is moved in direction **C,** thereby inserting it into septum 306a and removing it from fluid communication with pump chamber 208, thereby sealing reservoir 12 from pump chamber 208. Preferably, this state follows the state shown in **FIG. 5A** such that pump chamber 208 is filled with liquid drug 10 prior to movement of the needles 204a, 204b to set up the device for delivery of the liquid drug 10 to patient interface 214. In this state, when plunger 210 is moved in direction **F,** liquid drug 10 is forced from pump chamber 208 to patient interface 214 via needle 204b, while liquid drug 10 is prevented from returning to reservoir 12, as indicated by the "X" in **FIG. 5B****.**

**FIG. 5C** shows a third state of this embodiment of the valve wherein needles 204a, 204b are both in fluid communication with pump chamber 208 at the same time. In this state, needles 204a, 204b are both moved in direction **D** by actuators 502a, 502b respectively to pull them from septa 306a, 306b, thereby placing both needles 204a, 204b in fluid communication with pump chamber 208. This state allows the use of gaseous sterilization techniques (such as introducing ethylene oxide to the interior of the pump) to sterilize the pump after manufacture and before the device is placed into use. This state also helps with priming of the pump and the clearing of any air within the fluid path between reservoir 12 and patient interface 214 during the priming of the pump in which liquid drug 10 is moved into the empty volumes of the pump, such as the lumina of needles 204a, 204b and at least a portion of the volume of pump chamber 208. Plunger 210 may be moved in direction **E** to create a suction within pump chamber 208 thereby clearing reservoir 12, patient interface 214 and needles 204a, 204b of any residual air or repeatedly back and forth between directions **E** and **F** to prime the pump.

Note that, in various embodiments of the invention, side slit needles may be utilized as opposed to needles having the lumen at the end to avoid clogging the lumen with portions of a septum which may become dislodged when a needle is pushed into the septum. Further, side slit needles create a better seal with the septum than needles having the lumen at the end.

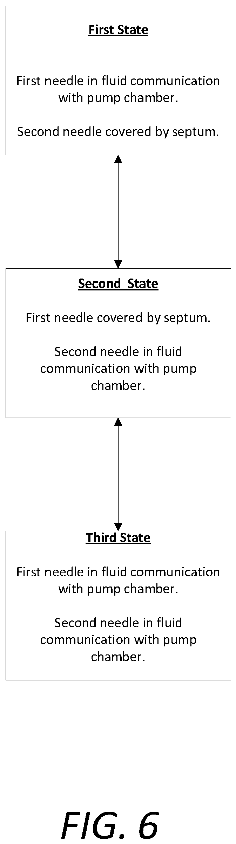

As shown in **FIG. 6****,** in various embodiments, the device may be in one of three states. In the first state, the first needle 204a, is in fluid communication with the reservoir 12, and is also in fluid communication with the pump chamber 208 and the second needle 204b, is in fluid communication with the patient interface, and is covered by a septum. The first state allows suction applied by the plunger 210 within pump chamber 208 to draw the liquid drug 10 from reservoir 12 into the pump chamber 208. In the second state, the first needle 204a is covered by a septum and the second needle 204b is in fluid communication with pump chamber 208.

The second state shown in **FIG. 6****,** the second needle 204b is ion fluid communication with pump chamber 208 allows pressure applied by plunger 210 within the pump chamber 208 to force the liquid drug 10 into the patient interface 214.

In the third state shown in **FIG. 6****,** both needles 204a, 204b are in fluid communication with the pump chamber 208, thereby enabling repeated back and forth motion of the plunger 210 in directions **E** and **F** to prime the pump (if reservoir 12 is filled with liquid drug 10) and to expunge any air from the fluid path between the reservoir 12 and the patient interface 214.

The following examples pertain to various embodiments of the invention:
Example 1 is a valve having a pump chamber, one or more exterior septa covering one or more openings in the pump chamber, a first needle and a second needle. The valve has two states wherein one or the other of the first and second needles may be placed in fluid communication with the pump chamber.
Example 2 is an extension of Example 1, or any other example disclosed herein, further comprising an actuator forming a mechanical coupling between the first and second needles.
Example 3 is an extension of Example 2, or any other example disclosed herein, wherein the actuator causes movement of the first and second needles in opposite linear directions.
Example 4 is an extension of Example 3, or any other example disclosed herein, wherein the actuator has a rotational movement that translates to linear movement of the first and second needles in opposite directions.
Example 5 is an extension of Example 2, or any other example disclosed herein, wherein the actuator comprises a shape memory alloy.
Example 6 is an extension of Example 3, or any other example disclosed herein, wherein the linear movement of the first and second needles in opposite directions causes one of the needles to be pushed out of a septum and into fluid communication with the pump chamber and the other needle to be pulled into a septum.
Example 7 is an extension of Example 4, wherein the valve further comprises one or more interior septa and wherein the linear movement of the first and second needles in opposite directions causes one of the needles to be pushed into an interior septum and the other needle to be pulled out of an interior septum and into fluid communication with the pump chamber.
Example 8 is an extension of Example 2, or any other example disclosed herein, wherein the actuator causes movement of the first and second needles in the same linear direction.
Example 9 is an extension of Example 8, wherein the first and second needles are of different lengths.
Example 10 is an extension of Example 9, or any other example disclosed herein, wherein the valve further comprises one or more interior septa and wherein linear movement of the first and second needles in a first direction causes one of the needles to be pushed into an interior septum and the other needle to be pushed out of an exterior septum and into fluid communication with the pump chamber.
Example 11 is an extension of Example 10, or any other example disclosed herein, wherein linear movement of the first and second needles in a second linear direction opposite the first linear direction causes one of the needles to be pulled into an exterior septum and the other needle to be pulled out of an interior septum and into fluid communication with the pump chamber.
Example 12 is an extension of Example 1, or any other example disclosed herein, wherein the lumen of one of the needles will be covered by a septum before the other needle is placed in fluid communication with the pump chamber.
Example 13 is an extension of Example 1, or any other example disclosed herein, wherein the first and second needles are both in fluid communication with the pump chamber.
Example 14 is an extension of Example 13, or any other example disclosed herein, wherein the first and second needles are able to be moved in first or second linear directions independently of each other.
Example 15 is extension of Example 1, or any other example disclosed herein, wherein the first needle is in fluid communication with a reservoir for containing a liquid drug and the second needle is in fluid communication with a patient interface.
Example 16 is an extension of Example 15, or any other example disclosed herein, wherein a plunger disposed within the pump chamber can alternately cause a suction within the pump chamber and a pressure within the pump chamber by moving in opposite directions.
Example 17 is an extension of Example 16, or any other example disclosed herein, wherein a suction within the pump chamber causes a liquid drug disposed in the reservoir to flow into the pump chamber.
Example 18 is an extension of Example 15, or any other example disclosed herein, wherein a pressure within the pump chamber causes the liquid drug in the pump chamber to flow to the patient interface.
Example 19 is an extension of Example 13, or any other example disclosed herein, wherein repeated back-and-forth motions to the plunger may be used to prime the pump and to expunge any air in fluid path between the reservoir and the patient interface.
Example 20 is an extension of Example 13, or any other example disclosed herein, further comprising first and second actuators coupled to the first and second needles respectively for linearly translating the first and second needles and wherein the first and second actuators are composed of a shape memory alloy.

Certain embodiments of the present invention were described above. It is, however, expressly noted that the present invention is not limited to those embodiments, but rather it is intended that additions and modifications to the expressly described embodiments herein are also to be included within the scope of the invention. Moreover, it is to be understood that the features of the various embodiments described herein were not mutually exclusive and can exist in various combinations and permutations, even if such combinations or permutations were not made express herein, without departing from the spirit and scope of the invention. As such, the invention is not to be defined only by the preceding illustrative description. Future filed applications claiming priority to this application may claim the disclosed subject matter in a different manner and may generally include any set of one or more limitations as variously disclosed or otherwise demonstrated herein.

Although the present invention is defined in the attached claims, it should be understood that the present invention can also (alternatively) be defined in accordance with the following embodiments:
1. A valve comprising:
   a pump chamber having one or more openings;
   one or more septa covering the one or more openings;
   a first needle; and
   a second needle;
   wherein, in a first state, a lumen of the first needle is in fluid communication with the pump chamber and a lumen of the second needle is covered by one of the one or more septa; and
   wherein, in a second state, the lumen of the second needle is in fluid communication with the pump chamber and the lumen of this first needle is covered by one of the one or more septa.
2. The valve of embodiment 1 further comprising:
   an actuator forming a mechanical coupling between the first and second needles.
3. The valve of one of embodiments 1 or 2 wherein the actuator causes movement of the first and second needles in opposite linear directions.
4. The valve of one of the preceding embodiments wherein the actuator has a rotational movement that translates to linear movement of the first and second needles in opposite directions.
5. The valve of one of the preceding embodiments wherein the actuator comprises a shape memory alloy wire.
6. The valve of one of the preceding embodiments wherein linear movement of the first and second needles in opposite linear directions causes one of the first and second needles to be pushed out of a septum and into fluid communication with the pump chamber and the other of the first and second needles to be pulled into a septum.
7. The valve of one of the preceding embodiments further comprising:
   one or more interior septa disposed on the interior of the pump chamber;
   wherein linear movement of the first and second needles in opposite linear directions causes one of the first and second needles to be pushed into an interior septum and the other of the first and
   second needles to be pulled out of an interior septum and into fluid communication with the pump chamber.
8. The valve of one of the preceding embodiments wherein the actuator causes movement of the first and second needles in the same linear direction.
9. The valve of one of the preceding embodiments wherein the first and second needles are of different lengths or are offset from each other with respect to the pump chamber.
10. The valve of one of the preceding embodiments further comprising:
   one or more interior septa disposed on the interior of the pump chamber;
   wherein linear movement of the first and second needles in a first linear direction causes one of the first and second needles to be pushed into an interior septum and the other of the first and
   second needles to be pushed out of an exterior septum and into fluid communication with the pump chamber.
11. The valve of one of the preceding embodiments wherein linear movement of the first and second needles in a second linear direction, opposite the first linear direction, causes one of the first and second needles to be pulled into an exterior septum and the other of the first and second needles to be pulled out of an interior septum and into fluid communication with the pump chamber.
12. The valve of one of the preceding embodiments wherein, during a transition between the first and second states, the lumen of one of the first and second needles will be covered by a septa before the other of the first and second needles is placed in fluid communication with the pump chamber.
13. The valve of one of the preceding embodiments wherein, in a third state, the lumina of the first and second needles are both in fluid communication with the pump chamber.
14. The valve of one of the preceding embodiments wherein the first and second needles are able to be moved in first or second linear directions independently of each other.
15. The valve of one of the preceding embodiments, further comprising:
   a first actuator coupled to the first needle for moving the first needle in first or second linear directions; and
   a second actuator coupled to the second needle for moving the second needle in first or second linear directions;
   wherein the first and second actuators comprise a shape memory alloy wire.
16. The valve of one of the preceding embodiments wherein:
   the first needle is in fluid communication with a reservoir for containing a liquid drug; and
   the second needle is in fluid communication with a patient interface.
17. The valve of one of the preceding embodiments further comprising:
   a plunger disposed within the pump chamber;
   wherein movement of the plunger in a first direction causes a suction within the pump chamber; and
   wherein movement of the plunger in a second direction, opposite the first direction, causes a pressure within the pump chamber.
18. The valve of one of the preceding embodiments wherein, in the first state, the suction within the pump chamber causes a liquid drug disposed in the reservoir to flow into the pump chamber via the first needle.
19. The valve of one of the preceding embodiments wherein, in the second state, the pressure within the pump chamber causes the liquid drug in the pump chamber to flow to the patient interface via the second needle.
20. The valve of one of the preceding embodiments wherein, in the third state, repeated back and forth motions of the plunger within the pump chamber may be used to prime the pump to expunge any air in a fluid path between the reservoir and the patient interface.

## Claims

1. A valve comprising:
a pump chamber having one or more openings;
one or more septa covering the one or more openings;
a first needle; and
a second needle;
wherein, in a first state, a lumen of the first needle is in fluid communication with the pump chamber and a lumen of the second needle is covered by one of the one or more septa; and
wherein, in a second state, the lumen of the second needle is in fluid communication with the pump chamber and the lumen of this first needle is covered by one of the one or more septa.

2. The valve of claim 1 further comprising:
an actuator forming a mechanical coupling between the first and second needles.

3. The valve of one of claims 1 or 2 wherein the actuator causes movement of the first and second needles in opposite linear directions.

4. The valve of one of the preceding claims wherein the actuator has a rotational movement that translates to linear movement of the first and second needles in opposite directions.

5. The valve of one of the preceding claims wherein the actuator comprises a shape memory alloy wire.

6. The valve of one of the preceding claims wherein linear movement of the first and second needles in opposite linear directions causes one of the first and second needles to be pushed out of a septum and into fluid communication with the pump chamber and the other of the first and second needles to be pulled into a septum.

7. The valve of one of the preceding claims further comprising:
one or more interior septa disposed on the interior of the pump chamber;
wherein linear movement of the first and second needles in opposite linear directions causes one of the first and second needles to be pushed into an interior septum and the other of the first and second needles to be pulled out of an interior septum and into fluid communication with the pump chamber.

8. The valve of one of the preceding claims wherein the actuator causes movement of the first and second needles in the same linear direction.

9. The valve of one of the preceding claims wherein the first and second needles are of different lengths or are offset from each other with respect to the pump chamber.

10. The valve of one of the preceding claims further comprising:
one or more interior septa disposed on the interior of the pump chamber;
wherein linear movement of the first and second needles in a first linear direction causes one of the first and second needles to be pushed into an interior septum and the other of the first and second needles to be pushed out of an exterior septum and into fluid communication with the pump chamber.

11. The valve of one of the preceding claims wherein linear movement of the first and second needles in a second linear direction, opposite the first linear direction, causes one of the first and second needles to be pulled into an exterior septum and the other of the first and second needles to be pulled out of an interior septum and into fluid communication with the pump chamber.

12. The valve of one of the preceding claims wherein, during a transition between the first and second states, the lumen of one of the first and second needles will be covered by a septa before the other of the first and second needles is placed in fluid communication with the pump chamber.

13. The valve of one of the preceding claims wherein, in a third state, the lumina of the first and second needles are both in fluid communication with the pump chamber.

14. The valve of one of the preceding claims wherein the first and second needles are able to be moved in first or second linear directions independently of each other.

15. The valve of one of the preceding claims, further comprising:
a first actuator coupled to the first needle for moving the first needle in first or second linear directions; and
a second actuator coupled to the second needle for moving the second needle in first or second linear directions;
wherein the first and second actuators comprise a shape memory alloy wire.
